(19) Europäisches Patentamt
European Patent Office
Office européen des brevets

(11) Veröffentlichungsnummer: **0 513 621 A1**

(12) **EUROPÄISCHE PATENTANMELDUNG**

(21) Anmeldenummer: **92107530.5**

(22) Anmeldetag: **04.05.92**

(51) Int. Cl.5: **C07D 249/12**, C07D 249/14, C07D 401/12, C07D 405/12, C07D 417/12, C07D 407/12, C07D 409/12, C07D 403/12, C07D 413/12, A01N 47/38

(30) Priorität: **14.05.91 DE 4115618**

(43) Veröffentlichungstag der Anmeldung: **19.11.92 Patentblatt 92/47**

(84) Benannte Vertragsstaaten: **BE CH DE DK ES FR GB IT LI NL**

(71) Anmelder: **BAYER AG**

**W-5090 Leverkusen 1 Bayerwerk(DE)**

(72) Erfinder: **Findeisen, Kurt, Prof. Dr.**
**Dünfelder Strasse 28**
**W-5090 Leverkusen 1(DE)**
Erfinder: **Kuhnt, Dietmar, Dr.**
**Körnerstrasse 5**
**W-5090 Leverkusen 1(DE)**
Erfinder: **Müller, Klaus-Helmut, Dr.**
**Bockhackstrasse 55**
**W-4000 Düsseldorf 13(DE)**
Erfinder: **Haug, Michael, Dr.**
**Fahner Weg 5**
**W-5060 Bergisch Gladbach 2(DE)**
Erfinder: **König, Klaus, Dr.**
**Zum Hahnenberg 40**
**W-5068 Odenthal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Substituierte Triazolinone.**

(57) Die Erfindung betrifft neue substituierte Triazolinone der allgemeinen Formel (I)

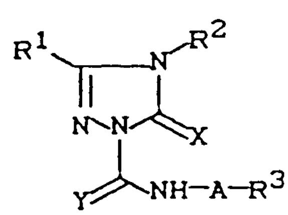

bei welchen
$R^1$ für einen Rest

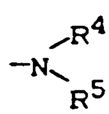

EP 0 513 621 A1

oder für einen Rest -S-$R^6$ steht,

$R^2$ für Alkyl steht,

$R^3$ für jeweils gegebenenfalls substituertes Cycloalkyl, Aryl oder Heterocyclyl steht,

A für einen der Reste

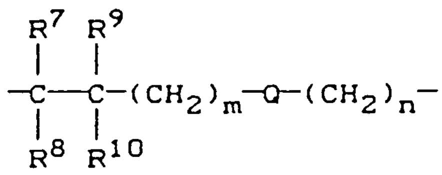 ; 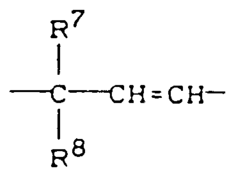 ;

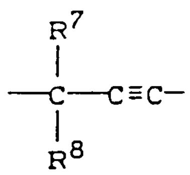 oder 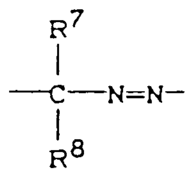 steht,

X für Sauerstoff oder Schwefel steht und

Y für Sauerstoff oder Schwefel steht,

wobei

$R^4$ für Wasserstoff oder Alkyl steht,

$R^5$ und $R^6$ unabhängig voneinander jeweils für Alkyl stehen,

$R^7$ entweder für Wasserstoff, Cyano oder Alkyl steht und

$R^8$, $R^9$, $R^{10}$ unabhängig voneinander jeweils für Wasserstoff oder Alkyl stehen oder

$R^7$ und $R^8$ gemeinsam für einen zweifach verknüpften Rest der Formel $-(CH_2)_p-$ stehen,

Q für Sauerstoff, Schwefel, eine Sulfinylgruppe, eine Sulfonylgruppe oder einen Rest der Formel #QAB#N-$R^{11}$ steht,

$R^{11}$ für Wasserstoff, Alkyl, Hydroxyalkyl, Alkoxyalkyl, Cyanalkyl oder Alkanoyl steht,

m für eine Zahl 0, 1 oder 2 steht und

p für eine Zahl 2, 3, 4, 5 oder 6 steht,

mehrere Verfahren Zu ihrer Herstellung und ihre Verwendung als Herbizide.

Die Erfindung betrifft neue substituierte Triazolinone, mehrere Verfahren zu ihrer Herstellung und ihre Verwendung als Herbizide.

Es ist bekannt, daß bestimmte substituierte Triazolinone wie beispielsweise die Verbindung 4-Methyl-3-methylamino-1-(4-phenylbut-2-ylaminocarbonyl)-1,2,4-triazolin-5-on oder die Verbindung 4-Methyl-3-dimethylamino-1-(4-phenylbut-2-yl-aminocarbonyl)-1,2,4-triazolin-5-on oder die Verbindung 4-Methyl-3-dimethylamino-1-[3-(4-trifluormethylphenyl)-prop-2-ylaminocarbonyl]-1,2,4-triazolin-5-on herbizide Eigenschaften besitzen (vergleiche z. B. EP 283 876 oder EP 398 096).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolinone der allgemeinen Formel (I) gefunden,

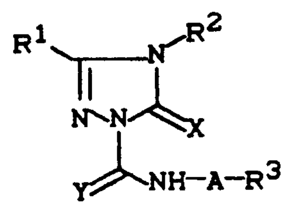

(I)

in welcher

$R^1$ für einen Rest

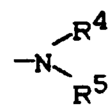

oder für einen Rest —S—$R^6$ steht,

$R^2$ für Alkyl steht,

$R^3$ für jeweils gegebenenfalls substituiertes Cycloalkyl, Aryl oder Heterocyclyl steht,

A für einen der Reste

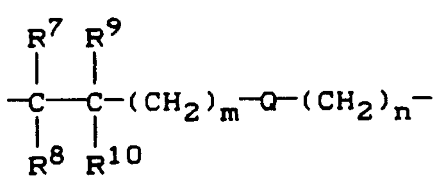 ; 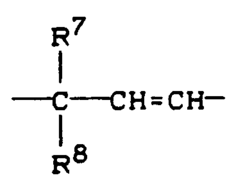 ;

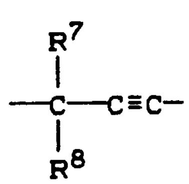 oder 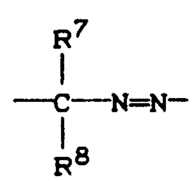

steht,

X für Sauerstoff oder Schwefel steht und

Y für Sauerstoff oder Schwefel steht,

wobei

$R^4$ für Wasserstoff oder Alkyl steht,

$R^5$ und $R^6$ unabhängig voneinander jeweils für Alkyl stehen,

$R^7$ entweder für Wasserstoff, Cyano oder Alkyl steht und

$R^8$, $R^9$ und $R^{10}$ unabhängig voneinander jeweils für Wasserstoff oder Alkyl stehen oder

$R^7$ und $R^8$ gemeinsam für einen zweifach verknüpften Rest der Formel $-(CH_2)_p-$ stehen,
Q für Sauerstoff, Schwefel, eine Sulfinylgruppe, eine Sulfonylgruppe oder einen Rest der Formel $>N-R^{11}$ steht,
$R^{11}$ für Wasserstoff, Alkyl, Hydroxyalkyl, Alkoxyalkyl, Cyanalkyl oder Alkanoyl steht,
m für eine Zahl 0, 1 oder 2 steht,
n für eine Zahl 0, 1 oder 2 steht und
p für eine Zahl 2, 3, 4, 5 oder 6 steht.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

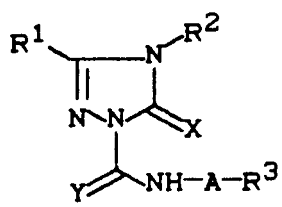

(I)

in welcher
$R^1$ für einen Rest

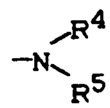

oder für einen Rest $-S-R^6$ steht,
$R^2$ für Alkyl steht,
$R^3$ für jeweils gegebenenfalls substituiertes Cycloalkyl, Aryl oder Heterocyclyl steht,
A für einen der Reste

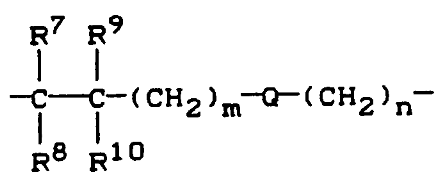 ; 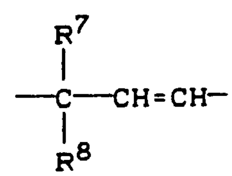 ;

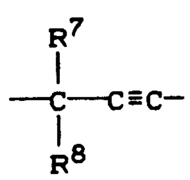 oder 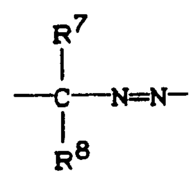

steht,
X für Sauerstoff oder Schwefel steht und
Y für Sauerstoff oder Schwefel steht,
wobei
$R^4$ für Wasserstoff oder Alkyl steht,
$R^5$ und $R^6$ unabhängig voneinander jeweils für Alkyl stehen,
$R^7$ entweder für Wasserstoff, Cyano oder Alkyl steht und
$R^8$, $R^9$ und $R^{10}$ unabhängig voneinander jeweils für Wasserstoff oder Alkyl stehen oder

| | |
|---|---|
| $R^7$ und $R^8$ | gemeinsam für einen zweifach verknüpften Rest der Formel $-(CH_2)_p-$ stehen, |
| Q | für Sauerstoff, Schwefel, eine Sulfinylgruppe, eine Sulfonylgruppe oder einen Rest der Formel $>N-R^{11}$ steht, |
| $R^{11}$ | für Wasserstoff, Alkyl, Hydroxyalkyl, Alkoxyalkyl, Cyanalkyl oder Alkanoyl steht, |
| m | für eine Zahl 0, 1 oder 2 steht, |
| n | für eine Zahl 0, 1 oder 2 steht und |
| p | für eine Zahl 2, 3, 4, 5 oder 6 steht, |

erhält, wenn man

a) 1-Chlor(thio)carbonyltriazolinone der Formel (II),

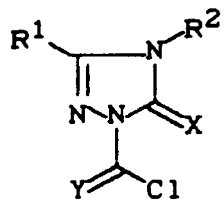

(II)

in welcher

$R^1$, $R^2$, X und Y die oben angegebene Bedeutung haben,

mit Aminen der Formel (III),

$H_2N—A—R^3$ (III)

in welcher

$R^3$ und A die oben angegebene Bedeutung haben,

gegebenenfalls in Gegenwart eines Verdünnungsmittels und gegebenenfalls in Gegenwart eines Reaktionshilfsmittels umsetzt,

oder wenn man

b) in 1-Stellung unsubstituierte Triazolinone der Formel (IV),

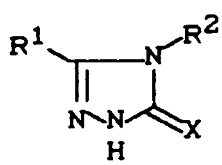

(IV)

in welcher

$R^1$, $R^2$ und X die oben angegebene Bedeutung haben,

mit Iso(thio)cyanaten der Formel (V),

$Y{=}C{=}N—A—R^3$ (V)

in welcher

$R^3$, A und Y die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazolinonen, wie beispielsweise die Verbindung 4-Methyl-3-methylamino-1-(4-phenylbut-2-yl-aminocarbonyl)-1,2,4-triazolin-5-on oder die Verbindung 4-Methyl-3-dimethylamino-1-(4-phenylbut-2-ylaminocarbonyl)-1,2,4-triazolin-5-on oder die Verbindung 4-Methyl-3-dimethylamino-1-[3-(4-trifluormethylphenyl)-prop-2-yl-aminocarbonyl]-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für einen Rest

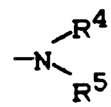

oder für einen Rest $-S-R^6$ steht,

$R^2$ für geradkettiges oder verzweigtes Alkyl mit 1 bis 6 Kohlenstoffatomen steht,

$R^3$ für gegebenenfalls einfach oder mehrfach, gleich oder verschieden substituiertes Cycloalkyl mit 3 bis 7 Kohlenstoffatomen steht, wobei als Substituenten infrage kommen: Halogen, Cyano, jeweils geradkettiges oder verzweigtes Alkyl mit 1 bis 4 Kohlenstoffatomen sowie jeweils geradkettiges oder verzweigtes Halogenalkyl mit jeweils 1 bis 4 Kohlenstoffatomen und 1 bis 9 gleichen oder verschiedenen Halogenatomen;

außerdem für jeweils gegebenenfalls einfach oder mehrfach, gleich oder verschieden substituiertes Aryl mit 6 oder 10 Kohlenstoffatomen oder Heteroaryl mit 2 bis 9 Kohlenstoffatomen und 1 bis 5 gleichen oder verschiedenen Heteroatomen - insbesondere Stickstoff, Sauerstoff und/oder Schwefel - steht, wobei als Substituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen substituiertes Phenyl, Phenoxy, $\alpha$-Naphthyl oder $\beta$-Naphthyl,

A für einen der Reste

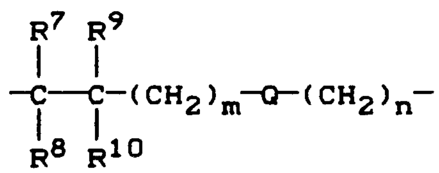 ; 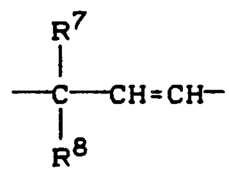 ;

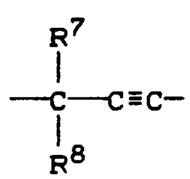 oder 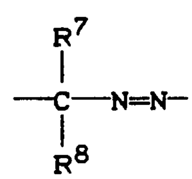

steht,

| | |
|---|---|
| X | für Sauerstoff oder Schwefel steht und |
| Y | für Sauerstoff oder Schwefel steht, wobei |
| $R^4$ | für Wasserstoff oder für geradkettiges oder verzweigtes Alkyl mit 1 bis 6 Kohlenstoffatomen steht, |
| $R^5$ und $R^6$ | unabhängig voneinander jeweils für geradkettiges oder verzweigtes Alkyl mit 1 bis 6 Kohlenstoffatomen stehen, |
| $R^7$ | entweder für Wasserstoff, Cyano oder für geradkettiges oder verzweigtes Alkyl mit 1 bis 8 Kohlenstoffatomen steht und |
| $R^8$, $R^9$ und $R^{10}$ | unabhängig voneinander jeweils für Wasserstoff oder für geradkettiges oder verzweigtes Alkyl mit 1 bis 6 Kohlenstoffatomen stehen oder |
| $R^7$ und $R^8$ | gemeinsam für einen zweifach verknüpften Rest der Formel $-(CH_2)_p-$ stehen, |
| Q | für Sauerstoff, Schwefel, eine Sulfinylgruppe, eine Sulfonylgruppe oder einen Rest der Formel $>N-R^{11}$ steht, |
| $R^{11}$ | für Wasserstoff oder für jeweils geradkettiges oder verzweigtes Alkyl, Hydroxyalkyl, Alkoxyalkyl, Cyanalkyl oder Alkanoyl mit jeweils 1 bis 8 Kohlenstoffatomen in den einzelnen Alkylteilen steht, |
| m | für eine Zahl 0, 1 oder 2 steht, |
| n | für eine Zahl 0, 1 oder 2 steht und |
| p | für eine Zahl 4, 5 oder 6 steht. |

Besonders bevorzugt sind Verbindungen der Formel (I), bei welchen

| | |
|---|---|
| $R^1$ | für einen Rest 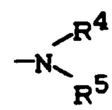 steht, |
| $R^2$ | für geradkettiges oder verzweigtes Alkyl mit 1 bis 4 Kohlenstoffatomen steht, |
| $R^3$ | für jeweils gegebenenfalls ein- bis fünffach, gleich oder verschieden substituiertes Cyclopropyl, Cyclobutyl, Cyclopentyl oder Cyclohexyl steht, wobei als Substituenten jeweils infrage kommen: Fluor, Chlor, Brom, Cyano, Methyl, Ethyl, n- oder i-Propyl, Chlormethyl, Dichlormethyl, Trichlormethyl, Difluormethyl oder Trifluormethyl; außerdem für jeweils gegebenenfalls ein- bis dreifach, gleich oder verschieden substituiertes Phenyl, $\alpha$-Naphthyl, $\beta$-Naphthyl, Indanyl, Pyridyl, Pyrimidyl, Triazinyl, Pyridazinyl, Pyrazolyl, Imidazolyl, Triazolyl, Thiazolyl, Isothiazolyl, Benzthiazolyl, Oxazolyl, Isoxazolyl, Benzoxazolyl, Benzopyrazolyl, Pyrrolyl, Furanyl, Thienyl, Indolyl, Benzopyranyl oder Chinolyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, $\alpha$-Naphthyl oder $\beta$-Naphthyl, |
| A | für einen der Reste |

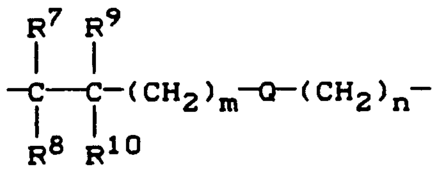 ; 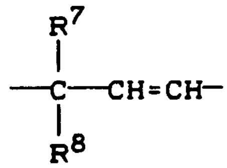 ;

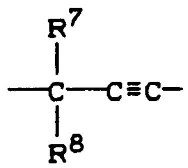 oder 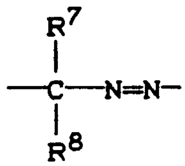

steht,

| | |
|---|---|
| X | für Sauerstoff oder Schwefel steht und |
| Y | für Sauerstoff oder Schwefel steht, |
| | wobei |
| $R^4$ | für Wasserstoff, Methyl oder Ethyl steht, |
| $R^5$ | für Methyl, Ethyl, n- oder i-Propyl steht, |
| $R^7$ | entweder für Wasserstoff, Cyano oder für geradkettiges oder verzweigtes Alkyl mit 1 bis 6 Kohlenstoffatomen steht und |
| $R^8$, $R^9$ und $R^{10}$ | unabhängig voneinander jeweils für Wasserstoff oder für geradkettiges oder verzweigtes Alkyl mit 1 bis 6 Kohlenstoffatomen stehen oder |
| $R^7$ und $R^8$ | gemeinsam für einen Rest der Formel $-(CH_2)_5-$ stehen, |
| Q | für Sauerstoff, Schwefel oder einen Rest der Formel $>N-R^{11}$ steht, |
| $R^{11}$ | für Wasserstoff oder für jeweils geradkettiges oder verzweigtes Alkyl, Hydroxyalkyl, Alkoxyalkyl, Cyanalkyl oder Alkanoyl mit jeweils 1 bis 4 Kohlenstoffatomen in den einzelnen Alkylteilen steht, |
| m | für eine Zahl 0 oder 1 steht und |
| n | für eine Zahl 0 oder 1 steht. |

Ganz besonders bevorzugt sind Verbindungen der Formel (I), bei welchen

| | |
|---|---|
| $R^1$ | für N-Methylamino, N-Ethylamino, N-i-Propylamino oder Dimethylamino steht, |
| $R^2$ | für Methyl, Ethyl, n- oder i-Propyl, n-, i-, s-oder t-Butyl steht, |
| $R^3$ | für gegebenenfalls ein- bis dreifach, gleich oder verschieden substituiertes Phenyl steht, wobei als Substituenten infrage kommen: |
| | Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, $\alpha$-Naphthyl oder $\beta$-Naphthyl, |
| A | für einen der Reste |

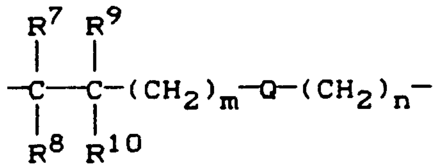 ; 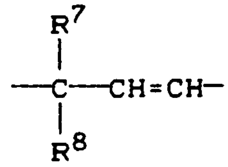 ;

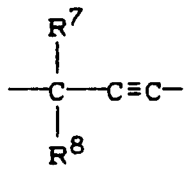 oder 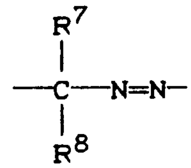

steht,

X für Sauerstoff steht und

Y für Sauerstoff steht,

wobei

$R^7$ entweder für Wasserstoff, Cyano oder für geradkettiges oder verzweigtes Alkyl mit 1 bis 4 Kohlenstoffatomen steht und

$R^8$, $R^9$ und $R^{10}$ unabhängig voneinander jeweils für Wasserstoff oder für geradkettiges oder verzweigtes Alkyl mit 1 bis 3 Kohlenstoffatomen stehen oder

$R^7$ und $R^8$ gemeinsam für einen Rest der Formel $-(CH_2)_5-$ stehen,

Q für Sauerstoff, Schwefel oder einen Rest der Formel $>N-R^{11}$ steht,

$R^{11}$ für Wasserstoff oder für jeweils geradkettiges oder verzweigtes Alkyl, Hydroxyalkyl, Alkoxyalkyl, Cyanalkyl oder Alkanoyl mit jeweils 1 bis 3 Kohlenstoffatomen in den einzelnen Alkylteilen steht,

m für eine Zahl 0 oder 1 steht und

n für eine Zahl 0 oder 1 steht.

Im einzelnen sei auf die bei den Herstellungsbeipielen aufgeführten Verbindungen verwiesen.

Verwendet man beispielsweise 1-Chlorcarbonyl-3-dimethylamino-4-methyl-1,2,4-triazolin-5-on und 1-(4-Methylphenoxy)-3-aminobutan als Ausgangsstoffe, so läßt sich der Reaktionsablauf des erfindungsgemäßen Verfahrens (a) durch das folgende Formelschema darstellen:

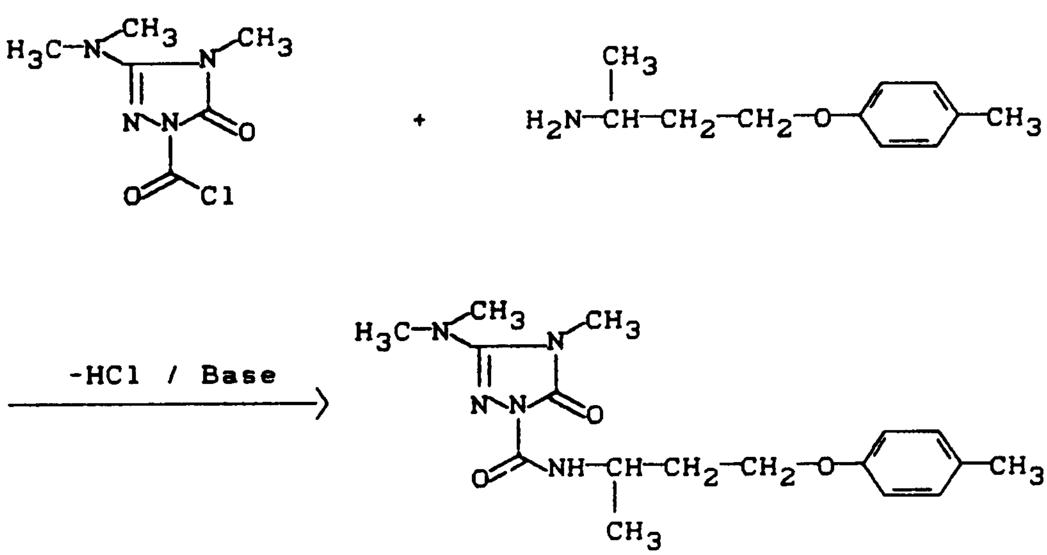


Verwendet man beispielsweise 3-Dimethylamino-4-methyl-1,2,4-triazolin-5-on und 1-Phenylazo-cyclohexylisocyanat als Ausgangsstoffe, so läßt sich der Reaktionsablauf des erfindungsgemäßen Verfahrens (b) durch das folgende Formelschema darstellen:

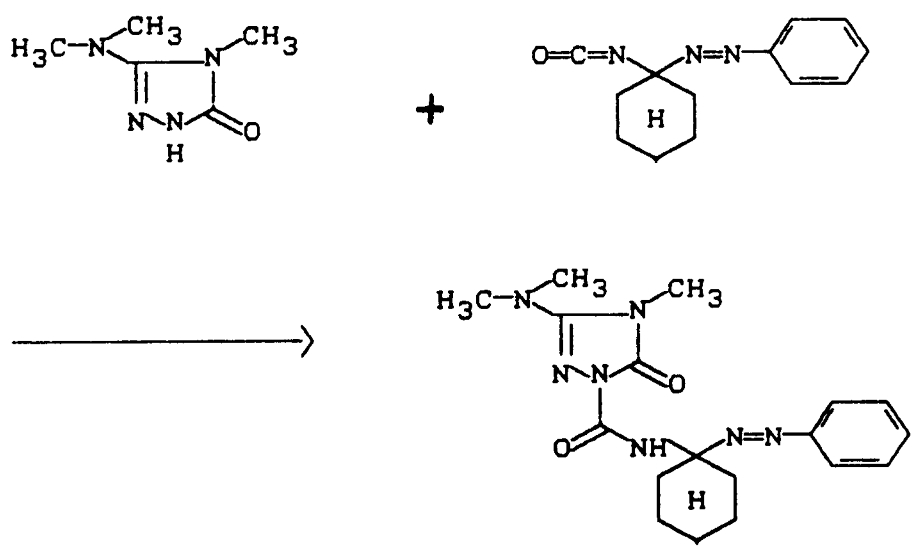

Die zur Durchführung des erfindungsgemäßen Verfahrens (a) als Ausgangsstoffe benötigten 1-Chlor(thio)carbonyltriazolinone sind durch die Formel (II) allgemein definiert. In dieser Formel (II) stehen $R^1$, $R^2$, X und Y vorzugsweise für diejenigen Reste, die bereits im Zusammenhang mit der Beschreibung der erfindungsgemäßen Verbindungen der Formel (I) als bevorzugt für diese Substituenten genannt wurden. Die 1-Chlor(thio)carbonyltriazolinone der Formel (II) sind bekannt (vergleiche z. B. EP 283 876).

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$ und A vorzugsweise für diejenigen Reste, die bereits im Zusammenhang mit der Beschreibung der erfindungsgemäßen Verbindungen der Formel (I) als bevorzugt für diese Substituenten genannt wurden.

Die Amine der Formel (III) sind bekannt oder erhältlich in Analogie zu bekannten Verfahren (vergleiche z. B. EP 273 328; Ind. J. Chem. Sect. B, 24B, 940-947 [1985]; Acta. Pharm. Suec., 20, 349-364 [1983] bzw. CA 100: 174345; An. Quim. 73, 1177-1183 [1977] bzw. CA 89: 129148; Bull. Soc. Chim. Belg. 85, 421-425 [1976]; Tetrahedron Lett. 1976, 2289-2290; Bull. Chem. Soc. Jpn. 60, 609-612 [1987]).

Die zur Durchführung des erfindungsgemäßen Verfahrens (b) als Ausgangsprodukte erforderlichen in 1-Stellung unsubstituierten Triazolinone sind durch die Formel (IV) allgemein definiert. In dieser Formel (IV) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolinone der Formel (IV) sind bekannt oder erhältlich in Analogie zu bekannten Verfahren (vergleiche z. B. EP 283 876).

Die zur Durchführung des erfindungsgemäßen Verfahrens (b) weiterhin als Ausgangsstoffe erforderlichen Iso(thio)cyanate sind durch die Formel (V) allgemein definiert. In dieser Formel (V) stehen $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (V) sind teilweise bekannt (vergleiche z. B. J. med. Chem. 30, 1767-1773 [1987]; J. org. Chem. 35, 47-52 [1970]; Sci. Pharm. 51, 379 [1983]; Synthesis 1984, 315; Synthesis 1990, 803; Angew. Chem. 98, 1111 [1986]; Nouv. J. Chim. 1. 243-254 [1977] bzw. CA 87: 151614a) oder erhältlich in Analogie zu bekannten Verfahren (vergleiche z. B. Synthesis 1977, 756; Org. Syntheses Coll. Vol. IV, 521 [1963] oder "Organikum" VEB Deutscher Verlag der Wissenschaften Berlin 1981, S. 703), beispielsweise, wenn man Amine der Formel (III),

$H_2N—A—R^3$ (III)

in welcher

$R^3$ und A die oben angegebene Bedeutung haben,

mit Phosgen oder Thiophosgen gegebenenfalls in Gegenwart eines Verdünnungsmittels, wie beispielsweise Toluol oder Chlorbenzol bei Temperaturen zwischen -10 °C und +150 °C umsetzt.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton, Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Basen wie Pyridin.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali- oder Erdalkalimetallacetate wie Natriumacetat, Kaliumacetat, Calciumacetat oder Ammoniumacetat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und +150 °C, vorzugsweise bei Temperaturen zwischen +10 °C und +80 °C.

Zur Durchführung des erfindungsgemäßen Verfahrens (a) setzt man pro Mol an 1-Chlor(thio)-carbonyltriazolinon der Formel (II) im allgemeinen 1.0 bis 5.0 Mol, vorzugsweise 1.0 bis 2 Mol an Amin der Formel (III) und gegebenenfalls 1.0 bis 5,0 Mol, vorzugsweise 1.0 bis 2 Mol an Reaktionshilfsmittel verwendeter Base ein.

Die Reaktionsdurchführung, Aufarbeitung und Isolierung der Reaktionsprodukte erfolgt nach bekannten Verfahren (vergleiche hierzu beispielsweise EP 283 876 oder die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) kann gegebenenfalls in Gegenwart eines geeigneten basischen Reaktionshilfsmittels durchgeführt werden. Als solche kommen alle üblichen anorganischen oder organischen Basen infrage. Vorzugsweise verwendet man tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Der Zusatz solcher basischer Reaktionshilfsmittel ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und +150 °C, vorzugsweise bei Temperaturen zwischen +40 °C und +120 °C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol an in 1-Stellung unsubstituiertem Triazolinon der Formel (IV) im allgemeinen 1,0 bis 5,0 Mol, vorzugsweise 1,0 bis 2,5 Mol an Iso(thio)cyanat der Formel (V) und gegebenenfalls 0,01 bis 5,0 Mol, vorzugsweise 0,1 bis 2,5 Mol an als Reaktionshilfsmittel verwendeter Base ein.

Die Reaktionsdurchführung, Aufarbeitung und Isolierung der Reaktionsprodukte erfolgt nach bekannten Verfahren (vergleiche hierzu beispielsweise EP 283 876 oder die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica,

Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen Kulturen wie beispielsweise Weizen oder Mais einsetzen. In entsprechenden Aufwandmengen besitzen die erfindungsgemäßen Wirkstoffe auch eine insektizide Wirksamkeit und lassen sich beispielsweise zur Bekämpfung von Reiszikaden einsetzen.

Die Verbindungen der Formel (I) versickern extrem langsam im Boden; somit ist eine Belastung des Grundwassers praktisch ausgeschlossen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid oder Dimethylsulfoxid sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung

mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); Methyl-6,6-dimethyl-2,4-dioxo-3-[1-(2-propenyloxyamino)-butylidenl-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(3-Chlorphenyl)-isopropylcarbamat (CHLORPROPHAM); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); 1-Methyl-3-phenyl-5-(3-trifluormethylphenyl)-4-pyridon(FLURIDONE); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-{[[((4-Methoxy-6-methyl-1,3,5-triazin-2-yl)-amino)-carbonyl]-amino]-sulfonyl}-benzoesäure oder deren Methylester (METSULFURON); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); Isopropyl-N-phenyl-carbamat (PROPHAM); 0-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) oder 2,6-Dinitro-4-trifluormethyl-N,N-dipropylanilin (TRIFLURALIN) sind gegebenenfalls von Vorteil. Einige Mischungen zeigen überraschenderweise auch synergistische Wirkungen.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen; Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Herstellungsbeispiele:

Beispiel 1

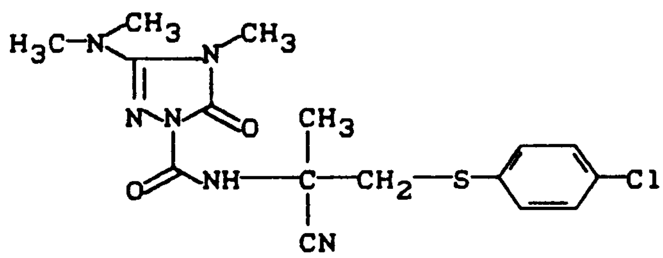

(Verfahren a)

Zu 6 g (0,03 Mol) 1-Chlorcarbonyl-3-dimethylamino-4-methyl-1,2,4-triazolin-5-on in 150 ml Acetonitril gibt man tropfenweise unter Rühren bei Raumtemperatur eine Lösung von 6,8 g (0,03 Mol) 2-Amino-1-(4-chlorphenylthio)-2-cyano-propan und 3,03 g (0,03 Mol) Triethylamin in 50 ml Acetonitril und rührt nach beendeter Zugabe weitere 2 Stunden bei Raumtemperatur. Zur Aufarbeitung wird ausgefallenes Triethylaminhydrochlorid abfiltriert, das Filtrat im Vakuum eingeengt, der Rückstand in 150 ml Dichlormethan aufgenommen, dreimal mit jeweils 50 ml Wasser gewaschen, über Natriumsulfat getrocknet und abermals im Vakuum eingeengt. Der Rückstand wird über Kieselgel chromatographiert (Laufmittel: Cyclohexan/Essigester 1:1).

Man erhält 6,8 g (58 % der Theorie) an 1-[1-(4-Chlorphenylthio)-2-cyanoprop-2-yl-aminocarbonyl]-3-dimethylamino-4-methyl-1,2,4-triazolin-5-on als Öl.

$^1$H-NMR ($CDCl_3$/Tetramethylsilan):

$\delta$ = 1,80; 2,85; 3,25; 7,15-7,20; 7,40-7,45; 8,25 ppm.

Herstellung der Ausgangsverbindung:

Beispiel III-1

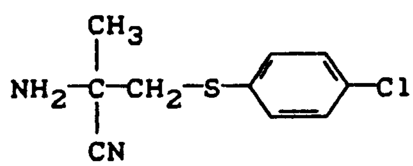

58,6 g (0,862 Mol) 25 %ige Ammoniaklösung und 9,6 g (0,179 Mol) Ammoniumchlorid werden zu einer Lösung von 8,79 g (0,179 Mol) Natriumcyanid in 13,8 ml Wasser gegeben. Anschließend gibt man 24,7 g (0,123 Mol) 1-(4-Chlorphenylthio)-2-propanon (käufliche Ware) zu und rührt dann 16 Stunden bei 45 °C. Zur Aufarbeitung wird das zweiphasige Reaktionsgemisch in Dichlormethan aufgenommen, mit Wasser gewaschen, über Natriumsulfat getrocknet und im Vakuum eingeengt.

Man erhält 26,5 g (95 % der Theorie) an 2-Amino-1-(4-chlorphenylthio)-2-propionitril als Öl.

IR: $\nu$ = 2200 (CN), 3250 ($NH_2$)$cm^{-1}$.

Beispiel 2

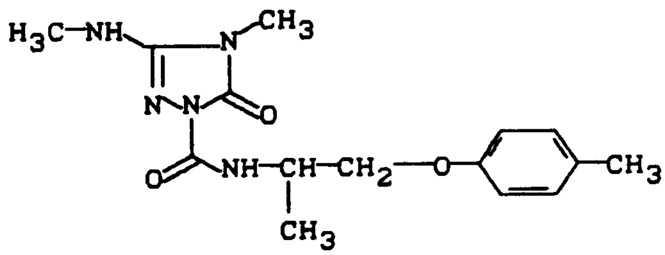

(Verfahren a)

Zu 3,81 g (0,02 Mol) 1-Chlorcarbonyl-3-methylamino-4-methyl-1,2,4-triazolin-5-on in 100 ml Acetonitril gibt man tropfenweise unter Rühren bei Raumtemperatur eine Lösung von 3,3 g (0,02 Mol) 2-Amino-1-(4-methylphenoxy)-propan (vergleiche z. B. EP 273 328) und 2,02 g (0,02 Mol) Triethylamin in 50 ml Acetonitril und rührt nach beendeter Zugabe weitere 2 Stunden bei Raumtemperatur. Zur Aufarbeitung wird ausgefallenes Triethylaminhydrochlorid abfiltriert, das Filtrat im Vakuum eingeengt, der Rückstand mit Wasser verrührt. Die dabei anfallenden Kristalle werden abfiltriert, mit Wasser gewaschen und getrocknet.

Man erhält 5,6 g (88 % der Theorie) an 1-[1-(4-Methylphenoxy)-prop-2-ylaminocarbonyl]-3-methylamino-4-methyl-1,2,4-triazolin-5-on vom Schmelzpunkt 96-98 °C (Zers.).

Beispiel 3

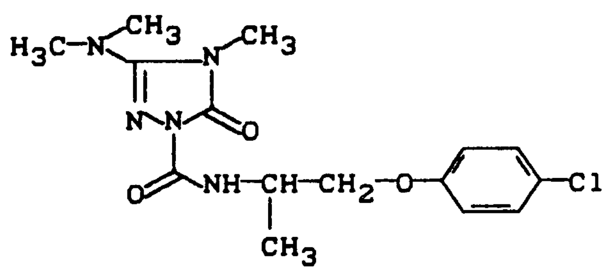

(Verfahren b)

Zu 4,26 g (0,03 Mol) 3-Dimethylamino-4-methyl-1,2,4-triazolin-5-on in 100 ml Acetonitril gibt man bei Raumtemperatur unter Rühren 6,33 g (0,03 Mol) 1-(4-Chlorphenoxy)-prop-2-ylisocyanat und 3 Tropfen 1,8-Diazabicyclo-[5.4.0]-undec-7-en (DBU), rührt anschließend 5 Stunden bei Raumtemperatur, engt dann im Vakuum ein und chromatographiert den Rückstand über Kieselgel (Laufmittel: Cyclohexan / Essigester 1:1).

Man erhält 8,9 g (84 % der Theorie) an 1-[1-(4-Chlorphenoxy)-prop-2-ylaminocarbonyl]-3-dimethylamino-4-methyl-1,2,4-triazolin-5-on als Öl.

$^1$H-NMR ($CDCl_3$/Tetramethylsilan): $\delta = 2{,}85; 3{,}25; 3{,}95\text{-}4{,}05; 8{,}10\text{-}8{,}15$ ppm.

Herstellung der Ausgangsverbindung

Beispiel V-1

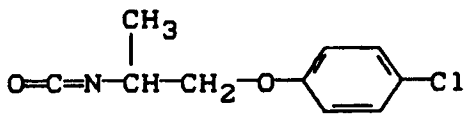

In 500 ml Chlorbenzol werden bei 0 ° C 340 g (3,4 Mol) Phosgen eingeleitet. Anschließend gibt man bei einer Temperatur zwischen 0 ° C und 25 ° C tropfenweise unter Rühren eine Lösung von 155 g (0,84 Mol) 1-(4-Chlorphenoxy)-2-propylamin (vergleiche z. B. Ind. J. Chem. Sect. B, 24B, 940-947 [1985]) in 200 ml Chlorbenzol zu, erwärmt unter weiterer Phosgeneinleitung langsam auf 90 ° C, bis die Lösung klar wird und rührt eine weitere Stunde bei 90 ° C nach. Zur Aufarbeitung wird das Lösungsmittel abdestilliert und der Rückstand im Vakuum destilliert.

Man erhält 112 g (65 % der Theorie) an 1-(4-Chlorphenoxy)-2-propylisocyanat vom Siedepunkt 120 ° C bei 0,5 mbar.

IR: $\nu$ = 2210 $cm^{-1}$ (N = C = O)

In entsprechender Weise und gemäß den allgemeinen Angaben zur Herstellung erhält man die folgenden substituierten Triazolinone der allgemeinen Formel (I):

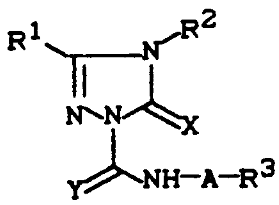

(I)

| Bsp.-Nr. | $R^1$ | $R^2$ | $R^3$ | A | X | Y | physikalische Eigenschaften |
|---|---|---|---|---|---|---|---|
| 4 | 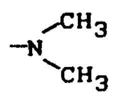 | $CH_3$ | 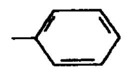 | 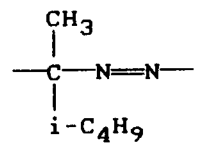 | O | O | $^1$H-NMR*): 0,68-0,7; 1,8; 2,85; 3,3; 9,72 |
| 5 | 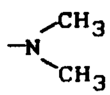 | $CH_3$ | 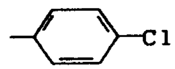 | 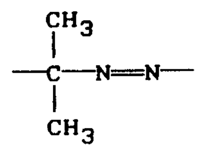 | O | O | $^1$H-NMR*): 1,81; 2,85; 3,3; 9,45 |
| 6 | 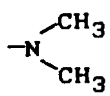 | $CH_3$ | 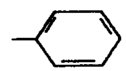 | 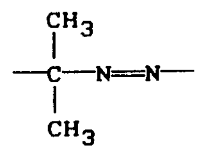 | O | O | Fp. 144-145° C |
| 7 | 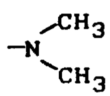 | $CH_3$ | 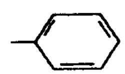 | 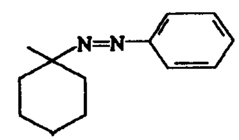 | O | O | $^1$H-NMR*): 1,5-2,5; 2,85; 3,3; 8,8 |
| 8 | 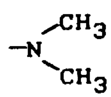 | $CH_3$ | 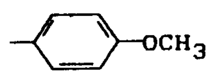 | 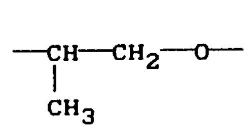 | O | S | $^1$H-NMR*): 1,45-1,48; 2,85; 3,25; 3,75; 4,95-5,05 |

| Bsp.-Nr. | $R^1$ | $R^2$ | $R^3$ | A | X | Y | physikalische Eigenschaften |
|---|---|---|---|---|---|---|---|
| 9 | 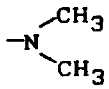 | $CH_3$ | 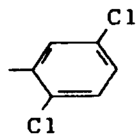 | 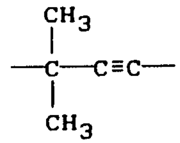 | O | O | $^1$H-NMR*): 1,81; 2,86; 3,26 |
| 10 | 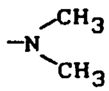 | $CH_3$ | 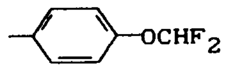 | 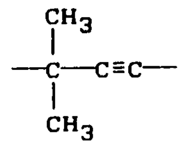 | O | O | $^1$H-NMR*): 1,78; 2,85; 3,25; 6,53 |
| 11 | 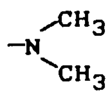 | $CH_3$ | 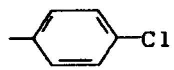 | 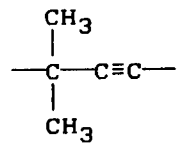 | O | O | $^1$H-NMR*): 1,78; 2,86 |
| 12 | 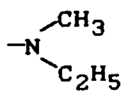 | $CH_3$ | 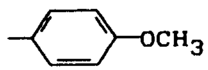 | 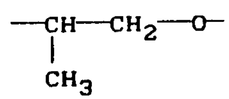 | O | O | $^1$H-NMR*): 1,17-1,25; 1,37-1,4; 2,85; 3,12-3,2; 3,75 |
| 13 | 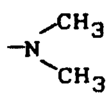 | $CH_3$ | 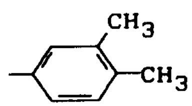 | 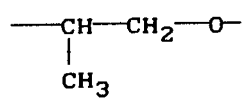 | O | O | $^1$H-NMR*): 1,48-1,5; 2,85; 3,25; 3,93-4,05 |

| Bsp.-Nr. | $R^1$ | $R^2$ | $R^3$ | A | X | Y | physikalische Eigenschaften |
|---|---|---|---|---|---|---|---|
| 14 | 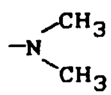 | $CH_3$ | 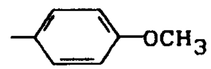 | 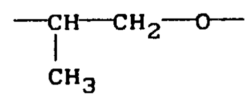 | O | O | $^1$H-NMR*): 1,48-1,5; 2,85; 3,25; 3,75; 3,92-4,05; 8,15-8,2 |
| 15 | $-NH-CH_3$ | $CH_3$ | 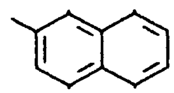 | $-CH_2-CH_2-NH-$ | O | O | Fp. 126-128° C |
| 16 | $-NH-CH_3$ | $CH_3$ | 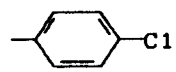 | 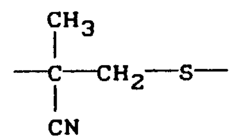 | O | O | Fp. 174-176° C |
| 17 | 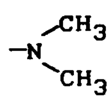 | $CH_3$ | 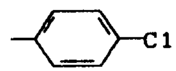 | 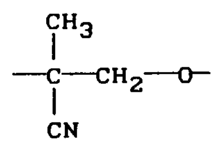 | O | O | Fp. 158-160° C |
| 18 | 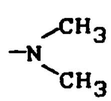 | $CH_3$ | 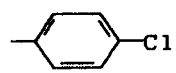 | 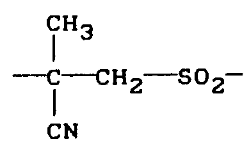 | O | O | Fp. 207-209° C |

| Bsp.-Nr. | $R^1$ | $R^2$ | $R^3$ | A | X | Y | physikalische Eigenschaften |
|---|---|---|---|---|---|---|---|
| 19 | $-NH-CH_3$ | $CH_3$ | 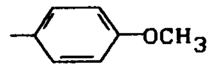 | 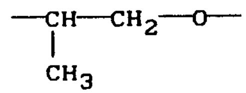 | O | O | Fp. 100-102° C |
| 20 | 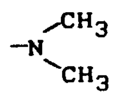 | $CH_3$ | 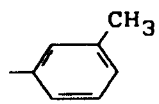 | 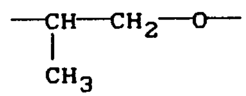 | O | O | ¹H-NMR*): 1,48-1,52; 2,3; 2,85; 4,35-4,45 |
| 21 | 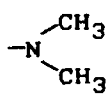 | $CH_3$ | 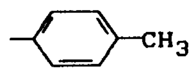 | 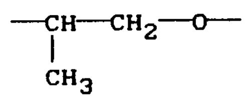 | O | O | ¹H-NMR*): 1,47-1,5; 2,28; 2,85; 3,25; 4,35-4,45; 8,15-8,2 |
| 22 | 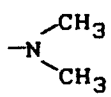 | $CH_3$ | 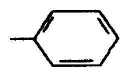 | 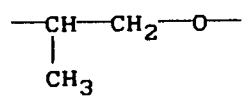 | O | O | ¹H-NMR*): 1,38-1,4; 2,85; 3,25; 8,1-8,15 |
| 23 | 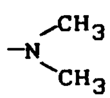 | $CH_3$ | 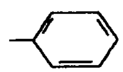 | 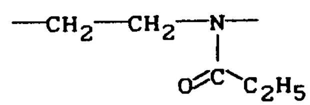 | O | O | Fp. 128-130° C |
| 24 | 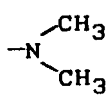 | $C_2H_5$ | 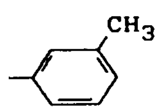 | 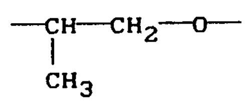 | O | O | ¹H-NMR*): 1,35-1,43; 2,32; 2,87; 3,68-3,75 |

| Bsp.-Nr. | $R^1$ | $R^2$ | $R^3$ | A | X | Y | physikalische Eigenschaften |
|---|---|---|---|---|---|---|---|
| 25 | 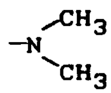 | $CH_3$ | 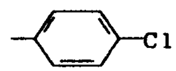 | 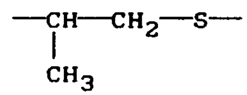 | O | O | $^1H$-NMR*): 1,35-1,38; 2,85; 3,25; 4,15-4,25 |
| 26 | 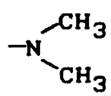 | $CH_3$ | 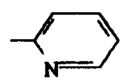 | 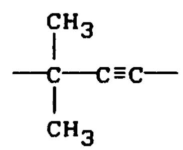 | O | O | $^1H$-NMR*): 1,82; 2,86; 3,26; 8,5-8,58 |
| 27 | 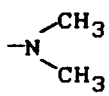 | $CH_3$ | 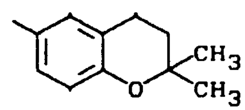 | 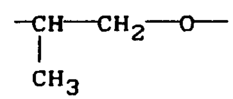 | O | O | $^1H$-NMR*): 1,3; 1,35-1,38; 2,85; 3,9-4,05; 4,3-4,45 |
| 28 | 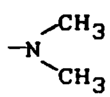 | $CH_3$ | 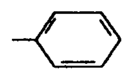 | 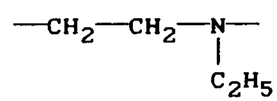 | O | O | $^1H$-NMR*): 1,13-1,18; 2,85; 3,25; 8,05-8,1 |
| 29 | 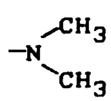 | $CH_3$ | 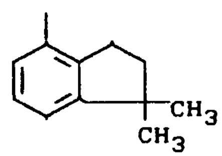 | 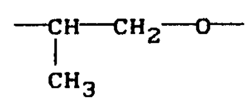 | O | O | $^1H$-NMR*): 1,25-1,28; 2,85; 3,25; 4,0-4,1 |

| Bsp.-Nr. | $R^1$ | $R^2$ | $R^3$ | A | X | Y | physikalische Eigenschaften |
|---|---|---|---|---|---|---|---|
| 30 | 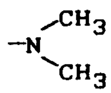 | $CH_3$ | 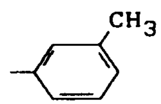 | 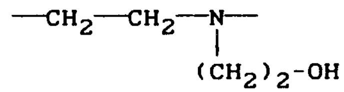 | O | O | $^1$H-NMR*): 1,85-1,95; 2,3; 2,85; 3,25; 3,4-3,5 |
| 31 | 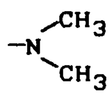 | $CH_3$ | 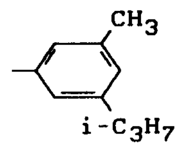 | 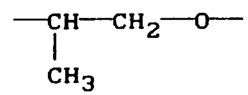 | O | O | $^1$H-NMR*): 1,2-1,23; 2,3; 2,85; 3,25; 6,58-6,65 |
| 32 | 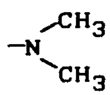 | $CH_3$ | 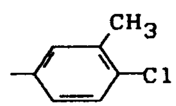 | 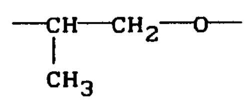 | O | O | $^1$H-NMR*): 1,48-1,52; 2,32; 2,85; 3,25; 8,15-8,2 |
| 33 | 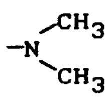 | $CH_3$ | 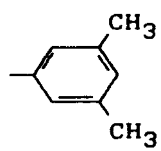 | 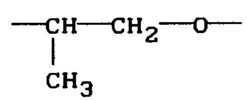 | O | O | $^1$H-NMR*): 1,47-1,5; 2,85; 3,25; 3,93-4,05 |
| 34 | 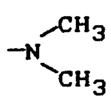 | $CH_3$ | 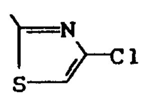 | 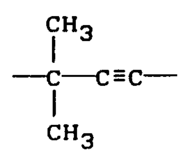 | O | O | Fp.: 72$^0$C |

| Bsp.-Nr. | $R^1$ | $R^2$ | $R^3$ | A | X | Y | physikalische Eigenschaften |
|---|---|---|---|---|---|---|---|
| 35 | $-S-CH_3$ | $CH_3$ | 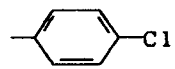 | 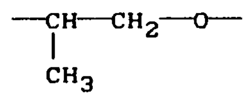 | O | O | Fp. 130-131° C |
| 36 | 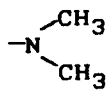 | $CH_3$ | 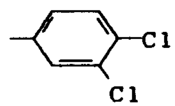 | $-CH_2-CH_2-S-CH_2-$ | O | O | ¹H-NMR*): 2,6-2,65; 2,85; 3,5-3,6; 7,15-7,45; 8,15-8,25 |
| 37 | 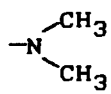 | $CH_3$ | 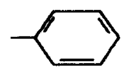 | $-CH_2-CH_2-CH_2-O-CH_2-$ | O | O | ¹H-NMR*): 1,87-1,95; 2,85; 3,25; 4,5; 7,35 |
| 38 | 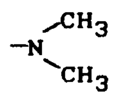 | $CH_3$ | 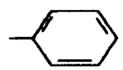 | 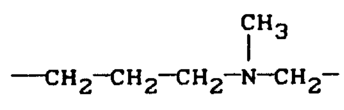 | O | O | ¹H-NMR*): 1,75-1,85; 2,2; 2,85; 3,25; 3,50 |
| 39 | 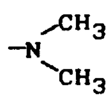 | $C_2H_5$ | 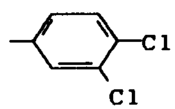 | $-CH_2-CH_2-CH_2-O-CH_2-$ | O | O | ¹H-NMR*): 1,33-1,4; 1,88-2,0; 2,85; 3,5-3,6; 3,65-3,75 |

| Bsp.-Nr. | $R^1$ | $R^2$ | $R^3$ | A | X | Y | physikalische Eigenschaften |
|---|---|---|---|---|---|---|---|
| 40 | 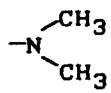 | $CH_3$ | 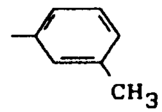 | $-CH_2-CH_2-CH_2-NH-$ | O | O | $^1$H-NMR*): 1,83-1,93; 2,28; 2,88; 3,25; 8,0-8,05 |
| 41 | 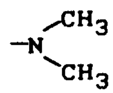 | $CH_3$ | 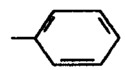 | $-CH_2-CH_2-CH_2-NH-$ | O | O | $^1$H-NMR*): 1,83-1,93; 2,85; 3,25; 3,47-3,53; 6,6-6,7 |
| 42 | 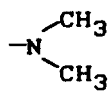 | $CH_3$ | 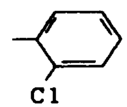 | $-CH_2-CH_2-CH_2-NH-$ | O | O | $^1$H-NMR*): 1,88-2,0; 2,85; 3,25; 3,5-3,55; 4,5,-4,55 |
| 43 | 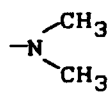 | $CH_3$ | 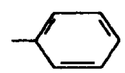 | 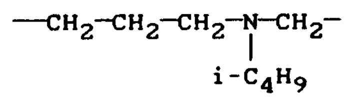 | O | O | $^1$H-NMR*): 0,88-0,9; 1,7-1,8; 2,85; 3,53; 7,17-7,35 |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

| Bsp.-Nr. | $R^1$ | $R^2$ | $R^3$ | A | X Y | physikalische Eigenschaften |
|---|---|---|---|---|---|---|
| 44 | 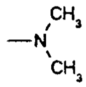 | $CH_3$ | 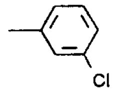 | 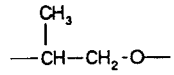 | O O | $^1$H-NMR*): 1,39-1,42; 2,88; 3,25; 4,35-4,45 |
| 45 | 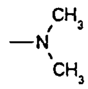 | $CH_3$ | 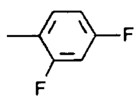 | 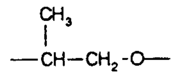 | O O | $^1$H-NMR*): 1,40-1,43; 2,88; 3,28; 4,32-4,42 |
| 46 | 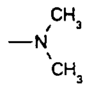 | $CH_3$ | 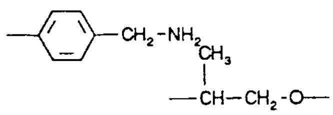 | | O O | $^1$H-NMR*): 1,40-1,42; 2,88; 3,25; 3,95-4,05; 4,5-4,52 |
| 47 | 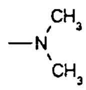 | $CH_3$ | 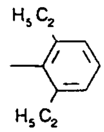 | $-CH_2-CH_2-NH-$ | O O | $^1$H-NMR*): 1,20-1,26; 2,62-2,70;2,85; 2,88; 3,58-3,65 |
| 48 | 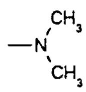 | $CH_3$ | 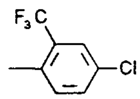 | $-CH_2-CH_2-NH-$ | O O | Fp. 135-137°C |
| 49 | 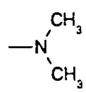 | $CH_3$ | 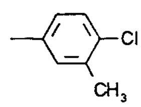 | $-CH_2-CH_2-NH-$ | O O | Fp. 144-146°C |
| 50 | 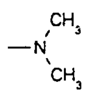 | $CH_3$ | 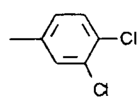 | $-CH_2-CH_2-NH-$ | O O | Fp. 146-148°C |

Anwendungsbeispiele:

In den folgenden Anwendungsbeispielen wurden die nachstehend aufgeführten Verbindungen als Vergleichssubstanzen eingesetzt:

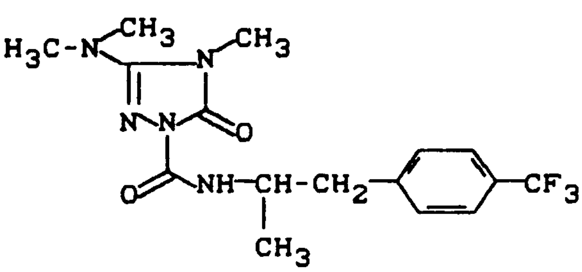

(A)

4-Methyl-3-dimethylamino-1-[3-(4-trifluormethylphenyl)-prop-2-ylaminocarbonyl]-1,2,4-triazolin-5-on

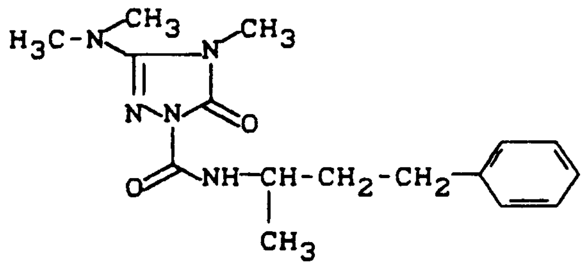

(B)

4-Methyl-3-dimethylamino-1-(4-phenylbut-2-ylaminocarbonyl)-1,2,4-triazolin-5-on

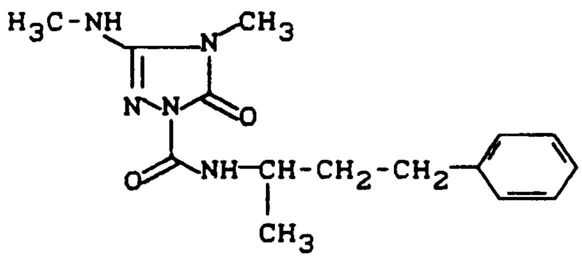

(C)

4-Methyl-3-methylamino-1-(4-phenylbut-2-ylaminocarbonyl)-1,2,4-triazolin-5-on
(alle bekannt aus EP 398 096)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 1 und 3.

**Patentansprüche**

**1.** Substituierte Triazolinone der allgemeinen Formel (I),

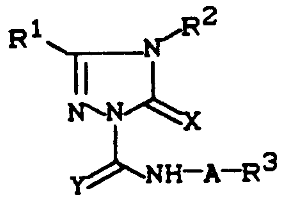

(I)

bei welchen

$R^1$ für einen Rest

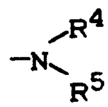

oder für einen Rest —S—$R^6$ steht,

$R^2$ für Alkyl steht,

$R^3$ für jeweils gegebenenfalls substituiertes Cycloalkyl, Aryl oder Heterocyclyl steht,

A für einen der Reste

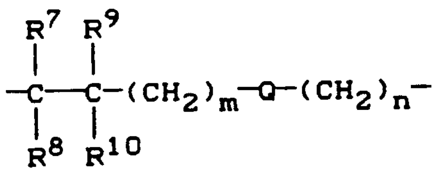 ; 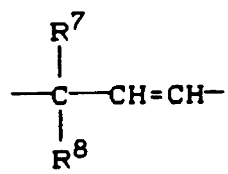 ;

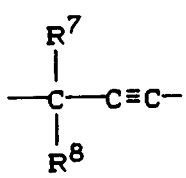 oder 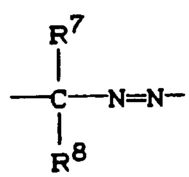

steht,

X für Sauerstoff oder Schwefel steht und

Y für Sauerstoff oder Schwefel steht,

wobei

$R^4$ für Wasserstoff oder Alkyl steht,

$R^5$ und $R^6$ unabhängig voneinander jeweils für Alkyl stehen,

$R^7$ entweder für Wasserstoff, Cyano oder Alkyl steht und

$R^8$, $R^9$ und $R^{10}$ unabhängig voneinander jeweils für Wasserstoff oder Alkyl stehen oder

$R^7$ und $R^8$ gemeinsam für einen zweifach verknüpften Rest der Formel $-(CH_2)_p$-stehen,

Q für Sauerstoff, Schwefel, eine Sulfinylgruppe, eine Sulfonylgruppe oder einen Rest der Formel $>$N-$R^{11}$ steht,

$R^{11}$ für Wasserstoff, Alkyl, Hydroxyalkyl, Alkoxyalkyl, Cyanalkyl oder Alkanoyl steht,

m für eine Zahl 0, 1 oder 2 steht,

n für eine Zahl 0, 1 oder 2 steht und

p für eine Zahl 2, 3, 4, 5 oder 6 steht.

**2.** Substituierte Triazolinone der allgemeinen Formel (I) gemäß Anspruch 1,

bei welchen

$R^1$ für einen Rest

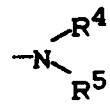

oder für einen Rest —S—$R^6$ steht,

$R^2$ für geradkettiges oder verzweigtes Alkyl mit 1 bis 6 Kohlenstoffatomen steht,

$R^3$ für gegebenenfalls einfach oder mehrfach, gleich oder verschieden substituiertes Cycloalkyl mit 3 bis 7 Kohlenstoffatomen steht, wobei als Substituenten infrage kommen:
Halogen, Cyano, jeweils geradkettiges oder verzweigtes Alkyl mit 1 bis 4 Kohlenstoffatomen sowie jeweils geradkettiges oder verzweigtes Halogenalkyl mit jeweils 1 bis 4 Kohlenstoffatomen und 1 bis 9 gleichen oder verschiedenen Halogenatomen;
außerdem für jeweils gegebenenfalls einfach oder mehrfach, gleich oder verschieden substituiertes Aryl mit 6 oder 10 Kohlenstoffatomen oder Heteroaryl mit 2 bis 9 Kohlenstoffatomen und 1 bis 5 gleichen oder verschiedenen Heteroatomen - insbesondere Stickstoff, Sauerstoff und/oder Schwefel - steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffstomen substituiertes Phenyl, Phenoxy, α-Naphthyl oder β-Naphtyl,

A für einen der Reste

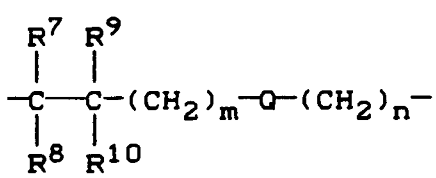 ; 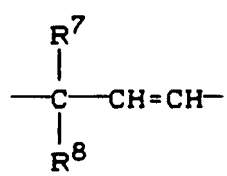 ;

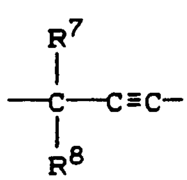 oder 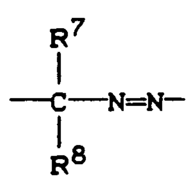

steht,

X für Sauerstoff oder Schwefel steht und

Y für Sauerstoff oder Schwefel steht,

wobei

$R^4$ für Wasserstoff oder für geradkettiges oder verzweigtes Alkyl mit 1 bis 6 Kohlenstoffatomen steht,

$R^5$ und $R^6$ unabhängig voneinander jeweils für geradkettiges oder verzweigtes Alkyl mit 1

bis 6 Kohlenstoffatomen stehen,

$R^7$ entweder für Wasserstoff, Cyano oder für geradkettiges oder verzweigtes Alkyl mit 1 bis 8 Kohlenstoffatomen steht und

$R^8$, $R^9$ und $R^{10}$ unabhängig voneinander jeweils für Wasserstoff oder für geradkettiges oder verzweigtes Alkyl mit 1 bis 6 Kohlenstoffatomen stehen oder

$R^7$ und $R^8$ gemeinsam für einen zweifach verknüpften Rest der Formel $-(CH_2)_p$-stehen,

Q für Sauerstoff, Schwefel, eine Sulfinylgruppe, eine Sulfonylgruppe oder einen Rest der Formel $>N-R^{11}$ steht,

$R^{11}$ für Wasserstoff oder für jeweils geradkettiges oder verzweigtes Alkyl, Hydroxyalkyl, Alkoxyalkyl, Cyanalkyl oder Alkanoyl mit jeweils 1 bis 8 Kohlenstoffatomen in den einzelnen Alkylteilen steht,

m für eine Zahl 0, 1 oder 2 steht,

n für eine Zahl 0, 1 oder 2 steht und

p für eine Zahl 4, 5 oder 6 steht.

**3.** Substituierte Triazolinone der allgemeinen Formel (I) gemäß Anspruch 1

bei welchen

$R^1$ für einen Rest

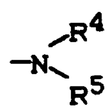

steht,

$R^2$ für geradkettiges oder verzweigtes Alkyl mit 1 bis 4 Kohlenstoffatomen steht,

$R^3$ für jeweils gegebenenfalls ein- bis fünffach, gleich oder verschieden substituiertes Cyclopropyl, Cyclobutyl, Cyclopentyl oder Cyclohexyl steht, wobei als Substituenten jeweils infrage kommen:

Fluor, Chlor, Brom, Cyano, Methyl, Ethyl, n-oder i-Propyl, Chlormethyl, Dichlormethyl, Trichlormethyl, Difluormethyl oder Trifluormethyl;

außerdem für jeweils gegebenenfalls ein-bis dreifach, gleich oder verschieden substituiertes Phenyl, $\alpha$-Naphthyl, $\beta$-Naphthyl, Indanyl, Pyridyl, Pyrimidyl, Triazinyl, Pyridazinyl, Pyrazolyl, Imidazolyl, Triazolyl, Thiazolyl, Isothiazolyl, Benzthiazolyl, Oxazolyl, Isoxazolyl, Benzoxazolyl, Benzopyrazolyl, Pyrrolyl, Furanyl, Thienyl, Indolyl, Benzopyranyl oder Chinolyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, $\alpha$-Naphthyl oder $\beta$-Naphthyl,

A für einen der Reste

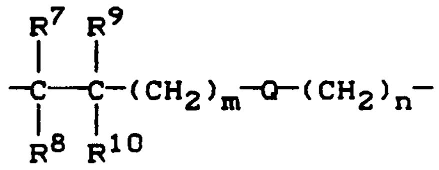 ; 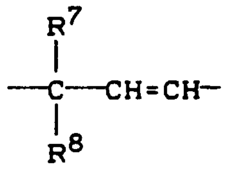 ;

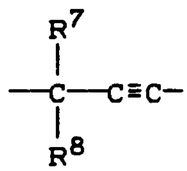 oder 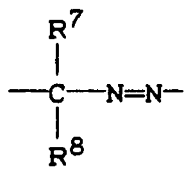

steht,

| | |
|---|---|
| X | für Sauerstoff oder Schwefel steht und |
| Y | für Sauerstoff oder Schwefel steht, wobei |
| $R^4$ | für Wasserstoff, Methyl oder Ethyl steht, |
| $R^5$ | für Methyl, Ethyl, n- oder i-Propyl steht, |
| $R^7$ | entweder für Wasserstoff, Cyano oder für geradkettiges oder verzweigtes Alkyl mit 1 bis 6 Kohlenstoffatomen steht und |
| $R^8$, $R^9$ und $R^{10}$ | unabhängig voneinander jeweils für Wasserstoff oder für geradkettiges oder verzweigtes Alkyl mit 1 bis 6 Kohlenstoffatomen stehen oder |
| $R^7$ und $R^8$ | gemeinsam für einen Rest der Formel $-(CH_2)_5-$ stehen, |
| Q | für Sauerstoff, Schwefel oder einen Rest der Formel $>N-R^{11}$ steht, |
| $R^{11}$ | für Wasserstoff oder für jeweils geradkettiges oder verzweigtes Alkyl, Hydroxyalkyl, Alkoxyalkyl, Cyanalkyl oder Alkanoyl mit jeweils 1 bis 4 Kohlenstoffatomen in den einzelnen Alkylteilen steht, |
| m | für eine Zahl 0 oder 1 steht und |
| n | für eine Zahl 0 oder 1 steht. |

**4.** Substituierte Triazolinone der allgemeinen Formel (I) gemäß Anspruch 1,

bei welchen

| | |
|---|---|
| $R^1$ | für N-Methylamino, N-Ethylamino, N-i-Propylamino oder Dimethylamino steht, |
| $R^2$ | für Methyl, Ethyl, n- oder i-Propyl, n-, i-, s- oder t-Butyl steht, |
| $R^3$ | für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl, |
| A | für einen der Reste |

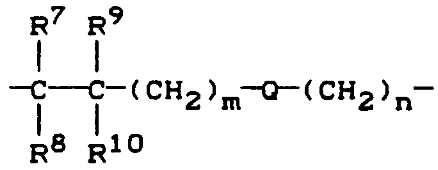 ; 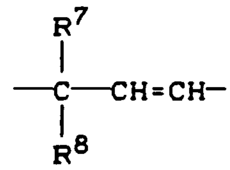 ;

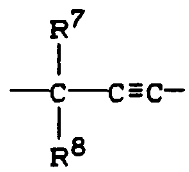 oder 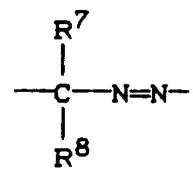

steht,

X für Sauerstoff steht und

Y für Sauerstoff steht, wobei

$R^7$ entweder für Wasserstoff, Cyano oder für geradkettiges oder verzweigtes Alkyl mit 1 bis 4 Kohlenstoffatomen steht und

$R^8$, $R^9$ und $R^{10}$ unabhängig voneinander jeweils für Wasserstoff oder für geradkettiges oder verzweigtes Alkyl mit 1 bis 3 Kohlenstoffatomen stehen oder

$R^7$ und $R^8$ gemeinsam für einen Rest der Formel $-(CH_2)_5-$ stehen,

Q für Sauerstoff, Schwefel oder einen Rest der Formel $>N-R^{11}$ steht,

$R^{11}$ für Wasserstoff oder für jeweils geradkettiges oder verzweigtes Alkyl, Hydroxyalkyl, Alkoxyalkyl, Cyanalkyl oder Alkanoyl mit jeweils 1 bis 3 Kohlenstoffatomen in den einzelnen Alkylteilen steht,

m für eine Zahl 0 oder 1 steht,

n für eine Zahl 0 oder 1 steht und

p für eine Zahl 2, 3, 4, 5 oder 6 steht.

**5.** Verfahren zur Herstellung von substituierten Triazolinonen der allgemeinen Formel (I) gemäß Anspruch 1,

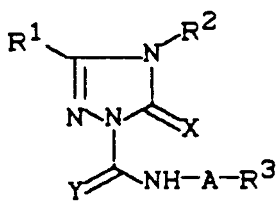 (I)

in welcher

$R^1$ für einen Rest

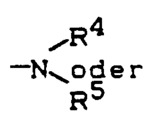 oder

für einen Rest $-S-R^6$ steht,

$R^2$ für Alkyl steht,

$R^3$ für jeweils gegebenenfalls substituiertes Cycloalkyl, Aryl oder Heterocyclyl steht,

A für einen der Reste

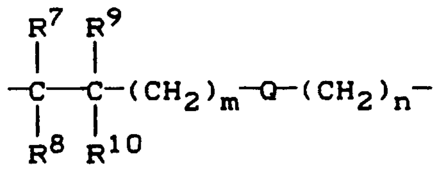 ; 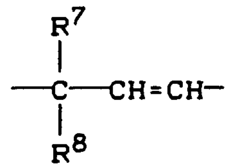 ;

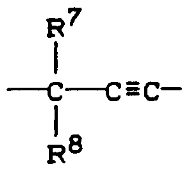 oder 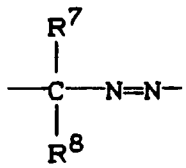

steht,

| | |
|---|---|
| X | für Sauerstoff oder Schwefel steht und |
| Y | für Sauerstoff oder Schwefel steht, wobei |
| $R^4$ | für Wasserstoff oder Alkyl steht, |
| $R^5$ und $R^6$ | unabhängig voneinander jeweils für Alkyl stehen, |
| $R^7$ | entweder für Wasserstoff, Cyano oder Alkyl steht und |
| $R^8$, $R^9$ und $R^{10}$ | unabhängig voneinander jeweils für Wasserstoff oder Alkyl stehen oder |
| $R^7$ und $R^8$ | gemeinsam für einen zweifach verknüpften Rest der Formel $-(CH_2)_p-$stehen, |
| Q | für Sauerstoff, Schwefel, eine Sulfinylgruppe, eine Sulfonylgruppe oder einen Rest der Formel $>N-R^{11}$ steht, |
| $R^{11}$ | für Wasserstoff, Alkyl, Hydroxyalkyl, Alkoxyalkyl, Cyanalkyl oder Alkanoyl steht, |
| m | für eine Zahl 0, 1 oder 2 steht, |
| n | für eine Zahl 0, 1 oder 2 steht und |
| p | für eine Zahl 2, 3, 4, 5 oder 6 steht, |

dadurch gekennzeichnet, daß man

a) 1-Chlor(thio)carbonyltriazolinone der Formel (II),

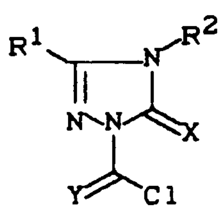

(II)

in welcher

$R^1$, $R^2$, X und Y die oben angegebene Bedeutung haben,

mit Aminen der Formel (III),

$H_2N—A—R^3$ (III)

in welcher

$R^3$ und A die oben angegebene Bedeutung haben,

gegebenenfalls in Gegenwart eines Verdünnungsmittels und gegebenenfalls in Gegenwart eines Reaktionshilfsmittels umsetzt,

oder daß man

b) in 1-Stellung unsubstituierte Triazolinone der Formel (IV),

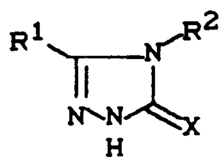

(IV)

in welcher

$R^1$, $R^2$ und X die oben angegebene Bedeutung haben,

mit Iso(thio)cyanaten der Formel (V),

$$Y{=}C{=}N{-}A{-}R^3 \qquad (V)$$

in welcher

$R^3$, A und Y die oben angegebene Bedeutung haben,

gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einem substituierten Triazolinon der Formel (I) gemäß den Ansprüchen 1 bis 5.

**7.** Verfahren zur Bekämpfung von unerwünschten Pflanzen, dadurch gekennzeichnet, daß man substituierte Triazolinone der Formel (I) gemäß den Ansprüchen 1 bis 5 auf unerwünschte Pflanzen und/oder ihren Lebensraum einwirken läßt.

**8.** Verwendung von substituierten Triazolinonen der Formel (I) gemäß den Ansprüchen 1 bis 5 zur Bekämpfung von unerwünschten Pflanzen.

**9.** Verfahren zur Herstellung von herbiziden Mitteln, dadurch gekennzeichnet, daß man substituierte Triazolinone der Formel (I) gemäß den Ansprüchen 1 bis 5 mit Streckmitteln und/oder oberflächenaktiven Substanzen vermischt.

Europäisches Patentamt

# EUROPÄISCHER RECHERCHENBERICHT

Nummer der Anmeldung

EP 92 10 7530

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-0 283 876 (BAYER AG)<br>* Ansprüche 1-8 * | 1,5-9 | C07D249/12<br>C07D249/14<br>C07D401/12<br>C07D405/12<br>C07D417/12<br>C07D407/12<br>C07D409/12<br>C07D403/12<br>C07D413/12<br>A01N47/38 |
| A | EP-A-0 399 294 (BAYER AG)<br>* Ansprüche 1-7; Beispiel 33 * | 1,5-9 | |
| A | EP-A-0 298 371 (BAYER AG)<br>* Ansprüche 1-8 * | 1,5-9 | |
| A,D | EP-A-0 398 096 (BAYER AG)<br>* Ansprüche 1-8 * | 1,5-9 | |
| A | EP-A-0 305 844 (BAYER AG)<br>* Ansprüche 1-7 * | 1,5-9 | |
| | | | RECHERCHIERTE SACHGEBIETE (Int. Cl.5 )<br>C07D<br>A01N |

Der vorliegende Recherchenbericht wurde für alle Patentansprüche erstellt

| Recherchenort | Abschlußdatum der Recherche | Prüfer |
|---|---|---|
| BERLIN | 14 AUGUST 1992 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)